# EUROPEAN PATENT SPECIFICATION

(11) **EP 2 670 909 B1**
(45) Date of publication and mention of the grant of the patent: **20.10.2021**
(21) Application number: 12742600.5
(22) Date of filing: 02.02.2012
(51) Int. Cl.: D21C 11/14, D21C 11/00, C07C 315/02, C07C 317/04

(54) **METHOD FOR PRODUCTION OF DIMETHYL SULFONE BY OXIDATION OF A METHANOL BASED MEDIUM OBTAINED FROM A SULFATE CELLULOSE PROCESS**
VERFAHREN ZUR HERSTELLUNG VON DIMETHYLSULFON DURCH OXIDATION EINES AUS EINEM SULFATCELLULOSEVERFAHREN GEWONNENEN MEDIUMS AUF METHANOLBASIS
PROCÉDÉ POUR LA PRODUCTION DE DIMÉTHYLSULFONE PAR OXYDATION D'UN MILIEU À BASE DE MÉTHANOL OBTENU À PARTIR D'UN PROCÉDÉ DE TRAITEMENT DE CELLULOSE AU SULFATE

(30) Priority: 03.02.2011 FI 20115109
(43) Date of publication of application: 11.12.2013
(73) Proprietor: Stora Enso Oyj, 00101 Helsinki (FI)
(72) Inventor: RÄSÄNEN, Tiina, 55420 Imatra (FI); VILJAMAA, Katja, 33180 Tampere (FI); ISOAHO, Jukka Pekka, 0740 Jyväskylä (FI); ALÉN, Raimo, 00520 Helsinki (FI); PAKKANEN, Hannu, 40600 Jyväskylä (FI)
(74) Representative: Berggren Oy
(86) International application number: PCT/FI2012/050101
(87) International publication number: WO 2012/104492

(56) References cited:
- WO-A1-94/05273
- US-A- 4 616 039
- US-A1- 2008 038 219
- US-A1- 2010 087 546
- US-A1- 2010 267 663
- US-A1- 2010 316 553
- US-B2- 6 552 231
- SAHLE-DEMESSIE E ET AL: "Oxidation of methanol and total reduced sulfur compounds with ozone over V2O5/TiO2 catalyst: Effect of humidity", APPLIED CATALYSIS A: GENERAL, ELSEVIER SCIENCE, AMSTERDAM, NL, vol. 361, no. 1-2, 20 June 2009 (2009-06-20), pages 72-80, XP026130250, ISSN: 0926-860X, DOI: 10.1016/J.APCATA.2009.03.035 [retrieved on 2009-04-05]
- Babak Karimi: "Supporting Information for: Selective Oxidation of Sulfides to Sulfoxides Using 30% Hydrogen Peroxide Catalyzed with a Recoverable Silica-Based Tungstate Interphase Catalyst", , 25 January 2005 (2005-01-25), XP55262176, Retrieved from the Internet: URL:http://pubs.acs.org/doi/suppl/10.1021/ ol047635d/suppl_file/ol047635dsi20050108_0 81557.pdf [retrieved on 2016-04-01]
- BOHME V H ET AL: "Zur Oxydation von Thioathern zu Sulfonen mit Wasserstoffperoxid in alkalischer Losung", CHEMIKER ZEITUNG, WILEY - V C H VERLAG GMBH & CO. KGAA, DE, vol. 96, no. 1-2, 1 January 1972 (1972-01-01), pages 36-38, XP008170236, ISSN: 0009-2894
- BABAK KARIMI ET AL: "Selective Oxidation of Sulfides to Sulfoxides Using 30% Hydrogen Peroxide Catalyzed with a Recoverable Silica-Based Tungstate Interphase Catalyst", ORGANIC LETTERS, vol. 7, no. 4, 1 February 2005 (2005-02-01), pages 625-628, XP055120742, ISSN: 1523-7060, DOI: 10.1021/ol047635d
- BOHME V.H. ET AL.: 'Zur Oxydation von Thioathern zu Sulfonen mit Wasserstoffperoxid in alkalischer Losung' CHEMIKER ZEITUNG vol. 96, no. 1-2, 1972, pages 37 - 38, XP008170236
- KARIMI, B. ET AL.: 'Selective oxidation of sulfides to sulfoxides using 30% hydrogen peroxide catalyzed with a recoverable silica-based tungstate interphase catalyst' ORGANIC LETTERS vol. 7, no. 4, 2005, pages 625 - 628, XP055120742
- Barbara Frunder ET AL: "Verzeichnis der Abkiirzungen" In: "Wörterbuch der Chemie", 1 June 1995 (1995-06-01), Deutscher Taschenbuch Verlag GmbH (dtv), DE, XP055589876, ISBN: 978-3-423-03360-2 pages 157-158,

## Description

The invention relates to a method for making dimethyl sulfone. Also disclosed is the use of dimethyl sulfone obtained by the method.

A prior known process of making dimethyl sulfone (CH₃-SO₂-CH₃) is the oxidation of dimethyl sulfoxide with hydrogen peroxide in water phase, which has been described in publication US 6,552,231 B2. At a high process temperature water is distilled off, which is made up for by adding water prior to crystallizing the end product.

Publication US 3,006,963 describes oxidation of various organic sulfides into respective sulfoxides and sulfones with an aqueous solution of hydrogen peroxide by means of a tungsten catalyst. Publication US 3,849,499 describes oxidations of organic sulfides, such as dibutyl sulfide, into sulfones in benzene or acetone medium. The oxidant is atmospheric oxygen and the catalysts are salts or oxides of metals, such as manganese, molybdenum or chromium.

The oxidation of dimethyl sulfide (CH₃-S-CH₃) into dimethyl sulfoxide (CH₃-SOCH₃) and further into dimethyl sulfone has been described e.g. in the article Yin, F., Grosjean, D., Flagan, R.C., Seinfeld, J.H., Journal of Atmospheric Chemistry II, 1990, pp. 365-399 as an atmospheric photochemical reaction. The preparation of dimethyl sulfone is disclosed also in Chemiker Zeitung, 1972, pp 36-38*.*

Various uses for dimethyl sulfone have been described, among others, in publications US 4,296,130, EP 0 200 526 B1, and US 6,552,231 B2. These include use as a skin and nail conditioner, a medical agent, a drug carrier, as well as a sulfur-containing dietary supplement.

An object of the invention is to provide a new way of making dimethyl sulfone, which offers a benefit of utilizing a starting material which consists of a cheap industrial by-product, thus far mostly regarded as hazardous waste. The invention is defined by the claims and is characterized in that dimethyl sulfone is prepared by oxidation from dimethyl sulfide in a methanol-based medium, that methanol is removed during and/or after the oxidation by distillation, water vapor distillation or evaporation, and that dimethyl sulfone is crystallized from a water-based solution phase.

Dimethyl sulfide is present as an ingredient in a liquid fraction obtained by liquefying a steam distillation product of a foul condensate from cooking vapours released in a sulfate pulp process and from a black liquor evaporation plant. The liquefied fraction mostly consists of methanol, and dimethyl sulfide is one of its sulfur contaminants causing odor nuisances.

The condensate has a general composition of > 50 weight-% (typically about 55 weight-%) of methanol, < 10 weight-% of ethanol, < 5 weight-% of acetone, about 10 weight-% of sulfur contaminants, and the balance (typically about 20 weight-%) is water. A typical composition of sulfur contaminants is in turn < 50 weight-% of methyl mercaptan, < 30 weight-% of dimethyl sulfide, < 20 weight-% of hydrogen sulfide, and < 10 weight-% of dimethyl disulfide.

The methanol-containing liquid fraction obtained from a sulfate pulp process has been exploited mostly by burning it in energy production. Fl patent publication 52710 describes a method for the purification of methanol removed from condensates of a sulfate process, wherein the condensate or impure methanol is treated with sulfuric acid, the methanol is distilled off of an aqueous phase, the along-distilled sulfur contaminants are oxidized with hydrogen peroxide, and the pure methanol is finally separated by further distillation. Oxidized sulfur compounds have not been described more closely in the publication. Neither is there any other knowledge of an attempt to separate sulfur components of the condensate from each other or to make any use of the same.

The invention is based on the surprising discovery that it is possible to oxidize dimethyl sulfide in a methanol-based solution phase into dimethyl sulfone, which can be crystallized from the solution selectively, such that other possibly present sulfur compounds are left in the solution. The boiling point of dimethyl sulfide is lower than that of methanol while the boiling point of dimethyl sulfone is higher than that of methanol, which means that, after the oxidation applied to a methanol-based solution, pure methanol is separable by evaporation, distillation or water vapor distillation, wherein the dimethyl sulfone is left in the evaporation or distillation residue.

When the process starting material is a liquefied fraction obtained from a sulfate pulp process, the cooking stages of the same, or from a black liquor evaporation plant, the oxidation converts also other low boiling sulfur contaminants of the condensate into higher boiling ones. Hence, methyl mercaptan has been found to oxidize supposedly into dimethyl sulfide and further into dimethyl sulfone in a way to improve the yield of the process. Oxidation also enables the separation of methanol by a single distillation or water vapor distillation cycle as pure methanol, which thus far has not been possible because of accompanying sulfur compounds. Therefore, an additional benefit of the invention is the capability of producing in a simple manner valuable pure methanol from a by-product of the sulfate pulp process, which until now has been burned and whose high sulfur content has been an environmental problem even in incineration.

The water vapor distillation of methanol provides the advantage of turning the liquid distillation residue, which contains the oxidized sulfur compounds, into a water-based residue, making dimethyl sulfone readily crystallizable as a pure end product. If necessary, water can be added to the distillation residue prior to the crystallization of dimethyl sulfone. The crystallization can take place by cooling the solution or evaporating water. If necessary, dimethyl sulfone can be further purified by recrystallization.

When the starting material is a liquefied methanol-containing fraction obtained from the sulfate pulp process, the water present in the fraction forms, upon the removal of methanol, an aqueous phase from which dimethyl sulfone is crystallizable. Thus, methanol can be removed by normal distillation or evaporation and the crystallization of dimethyl sulfone is achieved without the addition of water. In industrial scale, however, water vapor distillation is the most efficient course of action.

The first oxidation process stage is the oxidation of dimethyl sulfide in a methanol-based reaction medium into dimethyl sulfoxide, which in the second process stage further oxidizes into dimethyl sulfone which is crystallizable from an aqueous solution to obtain the end product. The formation of dimethyl sulfoxide in the first, strongly exothermic stage of the process is fast, while the further reaction in the second process stage into dimethyl sulfone is slow. The further reaction nevertheless speeds up considerably in response to heating the reaction medium. If the separation of methanol from the reaction medium is commenced prior to completing the oxidation process, conveniently while the second process stage is still in progress, the distillation brings heat to the reaction medium, which at the same time preferably accelerates the formation of dimethyl sulfone and thereby the completion of the process. Hence, whenever necessary, the supply of an oxidant into the reaction mixture can be continued even during the distillation.

Oxidants suitable for the invention include peroxides, oxygen, ozone, chlorine, permanganate or hypochlorite, especially hydrogen peroxide. The oxidation proceeds most favorably by making use of a metal-containing catalyst, although the oxidation has been found to work even without a catalyst. The metal contained in the catalyst can be Fe, Cu or Zn, in an elemental state or in the form of a compound, such as oxide or sulfate. If a catalyst is employed, it will be removed by filtration prior to the crystallization of dimethyl sulfone. One of the benefits of a catalyst is that, as by-products of the process, there may evolve small amounts of sulfur-containing inorganic oxidation products, such as sulfate, which adsorb to the catalyst and are removed with it.

Disclosed is that when the production starting material is a liquefied fraction obtained from a sulfate pulp process, which contains methanol as well as dimethyl sulfide, dimethyl disulfide and other sulfur contaminants, the fraction can be first subjected to oxidation, which is followed by the separation of methanol by distillation or water vapor distillation, whereby the sulfur-containing oxidation product is left in the distillation residue. A benefit of water vapor distillation is a water-based distillation residue from which dimethyl sulfone is readily crystallizable as a pure compound.

In the present invention said fraction is first subjected to distillation or water vapor distillation, whereby dimethyl sulfide is left in distillation residue; then the distillate is subjected to oxidation for oxidizing the dimethyl sulfide that has become distilled along with methanol; thereafter methanol is separated from the oxidation product of the preceding stage by distillation or water vapor distillation; and finally dimethyl sulfone is crystallized from the distillation residue left over from the separation of methanol. This procedure enables easy control of the amount of organic sulfur at oxidation and crystallization stages in the industrial scale process.

Also disclosed is the use of dimethyl sulfone produced as described above comprises the inclusion of dimethyl sulfone as an organic sulfur component in an orally ingested health formulation. Such formulations include e.g. a powder, which may consist solely of dimethyl sulfone, or tablets. Further disclosed is the use of dimethyl sulfone in an externally used therapeutic formulation. Such products include, among others, ointments applied to the skin, as well as eye and ear drops.

### Example 1

For the trials, in order to ensure comparability of the results, there was prepared a synthetic liquid mixture imitating a liquefied fraction obtained from a sulfate pulp process, with a constant composition of methanol (67.3 volume-%), water (19.2 volume-%), ethanol (6.7 volume-%), and acetone (2.9 volume-%), as well as sulfur compounds dimethyl sulfide (2.9 volume-%) and dimethyl disulfide (1.0 volume-%). 25 mL of this condensate (pH 7.5) was measured into an Erlenmeyer (100 mL), to which was then added 0.72 g of metallic iron as a catalyst and 6 mL of 30 volume-% hydrogen peroxide as an oxidant. The resulting mixture was stirred at room temperature for one hour, allowed to stabilize for 10 minutes without stirring, and filtered (filter's pore size 0.45 µm) for removing the catalyst. In order to demonstrate the oxidation product's crystallization effect, the mixture's methanol was allowed to evaporate gradually at room temperature at normal pressure, thus producing white crystals, which by a detailed X-ray diffraction analysis (D.E. Sands; Z. Kristallogr., Kristallgeom., Kristallphys., Kristallchem., 119(1963)245, D.A. Langs, J.V. Silverton and W.M. Bright; J. Chem. Soc. D., (1970)1653) were found to consist solely of pure dimethyl sulfone. On the other hand, the overall sulfur balance based on X-ray fluorescence measurement showed that the total yield of crystallized dimethyl sulfone was about 85 weight-% as calculated from initial sulfur. The negligible presence of inorganic sulfur compounds was confirmed ion chromatographically.

### Example 2

Oxidation of a synthetic mixture was conducted as in example 1, the only difference being that metal catalyst was not added to the mixture. Even in this case, the overall yield of dimethyl sulfone was found to remain at the level of 85 weight-%.

### Example 3

Oxidations of a synthetic mixture were conducted as in examples 1 and 2 with the difference that, prior to the oxidation, the pH of the mixture was adjusted with an aqueous solution of 25% sodium hydroxide to the value of 12. Even now, the total yield of dimethyl sulfone was about 85 weight-% in trials conducted both with catalysts and without a catalyst.

## Claims

1. A method for producing dimethyl sulfone, **characterized in that** the method comprises:
(a) providing a starting material for the production, which is a liquefied fraction obtained from a sulfate pulp process, said fraction having methanol as its main component and containing dimethyl sulfide, dimethyl disulfide and other sulfur contaminants;
(b) the fraction is subjected to distillation or water vapor distillation, whereby dimethyl disulfide is left in distillation residue;
(c) the distillate is subjected to oxidation for oxidizing the dimethyl sulfide that has become distilled along with methanol;
(d) methanol is separated from the oxidation product of step (c) by evaporation, distillation or water vapor distillation; and
(e) dimethyl sulfone is crystallized from the distillation residue of step (d).

2. A method as set forth in claim 1, **characterized in that** the oxidant is peroxide, oxygen, ozone, chlorine, permanganate, or hypochlorite, preferably hydrogen peroxide.

3. A method as set forth in any of the preceding claims, **characterized in that**, during the separation of methanol carried out by distillation or water vapor distillation, an oxidant, such as hydrogen peroxide, oxygen or air, is added to the reaction medium for carrying the oxidation to completion.

4. A method as set forth in any of the preceding claims, **characterized in that** water is added to the distillation residue, left over from the separation of methanol, prior to the crystallization of dimethyl sulfone.

5. A method as set forth in any of the preceding claims, **characterized in that** the crystallization is achieved by cooling the solution.

6. A method as set forth in any of the preceding claims, **characterized in that** the oxidation takes place in the presence of a metal-containing catalyst, and that the catalyst is removed by filtration prior to the crystallization of dimethyl sulfone.

7. A method as set forth in claim 6, **characterized in that** the metal contained in the catalyst is Fe, Cu or Zn, in an elemental state or in the form of a compound, such as oxide or sulfate.

8. A method as set forth in any of claims 1 to 7, **characterized in that** the starting material is containing methanol, water and a combination of sulfur contaminants which sulfur contaminants contain at most 50 wt-% of methyl mercaptan, at most 30 wt-% of dimethyl sulfide, at most 20 wt-% of hydrogen sulfide, and at most 10 wt-% of dimethyl disulfide based on the weight of the sulfur contaminants.

## Patentansprüche

1. Verfahren zur Herstellung von Dimethylsulfon, **dadurch gekennzeichnet, dass** das Verfahren Folgendes umfasst:
(a) Bereitstellen eines Ausgangsmaterials für die Herstellung, das eine verflüssigte Fraktion ist, die aus einem Sulfatzellstoffverfahren erhalten wird, wobei die Fraktion Methanol als Hauptkomponente aufweist und Dimethylsulfid, Dimethyldisulfid und andere Schwefelverunreinigungen enthält;
(b) die Fraktion einer Destillation oder Wasserdampfdestillation unterzogen wird, wobei Dimethyldisulfid im Destillationsrückstand zurückbleibt;
(c) das Destillat einer Oxidation unterzogen wird, um das zusammen mit Methanol destillierte Dimethylsulfid zu oxidieren;
(d) Methanol von dem Oxidationsprodukt von Schritt (c) durch Eindampfen, Destillation oder Wasserdampfdestillation abgetrennt wird; und
(e) Dimethylsulfon aus dem Destillationsrückstand von Schritt (d) kristallisiert wird.

2. Verfahren nach Anspruch 1, **dadurch gekennzeichnet, dass** das Oxidationsmittel Peroxid, Sauerstoff, Ozon, Chlor, Permanganat oder Hypochlorit, vorzugsweise Wasserstoffperoxid, ist.

3. Verfahren nach einem der vorhergehenden Ansprüche, **dadurch gekennzeichnet, dass** bei der destillativen oder wasserdampfdestillativen Abtrennung von Methanol dem Reaktionsmedium ein Oxidationsmittel wie Wasserstoffperoxid, Sauerstoff oder Luft zugesetzt wird, um die Oxidation zu Ende zu führen.

4. Verfahren nach einem der vorhergehenden Ansprüche, **dadurch gekennzeichnet, dass** dem bei der Methanolabtrennung anfallenden Destillationsrückstand vor der Kristallisation von Dimethylsulfon Wasser zugesetzt wird.

5. Verfahren nach einem der vorhergehenden Ansprüche, **dadurch gekennzeichnet, dass** die Kristallisation durch Abkühlen der Lösung erreicht wird.

6. Verfahren nach einem der vorhergehenden Ansprüche, **dadurch gekennzeichnet, dass** die Oxidation in Gegenwart eines metallhaltigen Katalysators erfolgt, und dass der Katalysator vor der Kristallisation von Dimethylsulfon durch Filtration entfernt wird.

7. Verfahren nach Anspruch 6, **dadurch gekennzeichnet, dass** das im Katalysator enthaltene Metall Fe, Cu oder Zn, in elementarem Zustand oder in Form einer Verbindung, wie Oxid oder Sulfat ist.

8. Verfahren nach einem der Ansprüche 1 bis 7, **dadurch gekennzeichnet, dass** das Ausgangsmaterial Methanol, Wasser und eine Kombination von Schwefelverunreinigungen enthält, wobei Schwefelverunreinigungen bezogen auf das Gewicht des Schwefelverunreinigungen höchstens 50 Gew.-% Methylmercaptan, höchstens 30 Gew.-% Dimethylsulfid, höchstens 20 Gew.-% Schwefelwasserstoff und höchstens 10 Gew.-% Dimethyldisulfid enthalten.

## Revendications

1. Procédé de production de diméthylsulfone, **caractérisé en ce que** le procédé comprend les étapes suivantes :
(a) la fourniture, pour la production, d'une matière de départ, qui est une fraction liquéfiée obtenue à partir d'un procédé de pâte au sulfate, ladite fraction ayant du méthanol comme composant principal et contenant du sulfure de diméthyle, du disulfure de diméthyle et d'autres contaminants soufrés ;
(b) la fraction est soumise à une distillation ou à une distillation à la vapeur d'eau, moyennant quoi le disulfure de diméthyle est laissé dans le résidu de distillation ;
(c) le distillat est soumis à une oxydation pour oxyder le sulfure de diméthyle distillé avec le méthanol ;
(d) le méthanol est séparé du produit d'oxydation de l'étape (c) par évaporation, distillation ou distillation à la vapeur d'eau ; et
(e) la diméthylsulfone est cristallisée à partir du résidu de distillation de l'étape (d).

2. Procédé selon la revendication 1, **caractérisé en ce que** l'oxydant est le peroxyde, l'oxygène, l'ozone, le chlore, le permanganate ou l'hypochlorite, de préférence le peroxyde d'hydrogène.

3. Procédé selon l'une quelconque des revendications précédentes, **caractérisé en ce que**, lors de la séparation du méthanol effectuée par distillation ou distillation à la vapeur d'eau, un oxydant, tel que du peroxyde d'hydrogène, de l'oxygène ou de l'air, est ajouté au milieu réactionnel pour amener l'oxydation à son terme.

4. Procédé selon l'une quelconque des revendications précédentes, **caractérisé en ce que** de l'eau est ajoutée au résidu de distillation, issu de la séparation du méthanol, avant la cristallisation de la diméthylsulfone.

5. Procédé selon l'une quelconque des revendications précédentes, **caractérisé en ce que** la cristallisation est réalisée en refroidissant la solution.

6. Procédé selon l'une quelconque des revendications précédentes, **caractérisé en ce que** l'oxydation a lieu en présence d'un catalyseur contenant du métal, et **en ce que** le catalyseur est éliminé par filtration avant la cristallisation de la diméthylsulfone.

7. Procédé selon la revendication 6, **caractérisé en ce que** le métal contenu dans le catalyseur est Fe, Cu ou Zn, à l'état élémentaire ou sous la forme d'un composé, tel qu'un oxyde ou un sulfate.

8. Procédé selon l'une quelconque des revendications 1 à 7, **caractérisé en ce que** la matière de départ contient du méthanol, de l'eau et une combinaison de contaminants soufrés, lesquels contaminants soufrés contiennent maximum 50 % en poids de méthylmercaptan, maximum 30 % en poids de sulfure de diméthyle, maximum 20 % en poids de sulfure d'hydrogène et maximum 10 % en poids de disulfure de diméthyle sur la base du poids des contaminants soufrés.
